# EUROPEAN PATENT SPECIFICATION

(11) **EP 3 684 395 B1**
(45) Date of publication and mention of the grant of the patent: **19.06.2024**
(21) Application number: 18769063.1
(22) Date of filing: 27.08.2018
(51) Int. Cl.: A61K 38/22, A61K 51/08, C07K 14/595, G01N 33/574

(54) **MINIGASTRIN DERIVATES, IN PARTICULAR FOR USE IN CCK2 RECEPTOR POSITIVE TUMOUR DIAGNOSIS AND/OR TREATMENT**
MINIGASTRINDERIVATE, INSBESONDERE ZUR VERWENDUNG IN POSITIVER CCK2-REZEPTOR-TUMORDIAGNOSE UND/ODER -BEHANDLUNG
DÉRIVÉS DE MINI-GASTRINE, EN PARTICULIER POUR UNE UTILISATION DANS LE DIAGNOSTIC ET/OU TRAITEMENT DE TUMEURS POSITIVES DU RÉCEPTEUR CCK2

(30) Priority: 21.09.2017 EP 17192428
(43) Date of publication of application: 29.07.2020
(73) Proprietor: Paul Scherrer Institut, 5232 Villigen PSI (CH)
(72) Inventor: BEHE, Martin, 4460 Gelterkinden (CH); SCHIBLI, Roger., 5400 Baden (CH); MINDT, Thomas., 1180 Wien (AT); GROB, Nathalie., 8057 Zürich (CH)
(74) Representative: Fischer, Michael
(86) International application number: PCT/EP2018/073020
(87) International publication number: WO 2019/057445

(56) References cited:
- WO-A1-2012/156511
- WO-A1-2015/067473
- US-A1- 2007 041 903
- LUDMILA MELICHAROVA ET AL: "Preclinical evaluation of gastrin derivatives labelled with <sup>111</sup>in: radiolabelling, affinity profile and pharmacokinetics in rats", BIOMEDICAL PAPERS OF THE FACULTY OF MEDICINE OF PALACK UNIVERSITY, OLOMOUC CZECH REPUBLIC, 27 September 2013 (2013-09-27), XP055453844, CZ ISSN: 1213-8118, DOI: 10.5507/bp.2013.064
- ROOSENBURG S ET AL: "PET and SPECT Imaging of a Radio labeled Minigastrin Analogue Conjugated with DOTA, NOTA, and NODAGA and Labeled with Cu-64, Ga-68, and In-111", MOLECULAR PHARMACEUTICS, vol. 11, no. 11, November 2014 (2014-11), pages 3930-3937, XP002778524, ISSN: 1543-8384

## Description

The present disclosure relates to minigastrin derivates and their use in CCK2 receptor positive tumour diagnosis and/or treatment.

G protein-coupled receptors (GPCR) are used as target proteins for radiolabelled peptides since the early 90's. The somatostatin receptor was the prototype for radionuclide imaging and therapy with peptides (Lit) resulting in a clinical first line therapies for neuroendocrine tumors with Y-90 and Lu-177 labelled derivatives of octreotide (Lit). Several radiolabelled peptides were tested for the possibility to target overexpressed GPCR on tumours including gastrin realising peptide analogues (GRP), glucagon-like peptide 1 analogues (GLP-1), neurotensin analogues (NT) or neuropeptide Y analogues (NPY) (Mäcke, Reubi J Nucl Med 2008; 49:1735-1738). An additional very interesting target is the cholecystokinin-2 receptor (CCK-2 R). This receptor is mainly expressed on medullary thyroid carcinomas (MTC), small cell lung cancers (SCLC) and stromal ovarial tumors (Reubi, Int J Cancer. 1996 and Reubi, Cancer Res. 1997). Radiolabelled gastrin analogues are good candidates for targeting imaging and therapy. It was shown that In-111 labelled gastrin analogues are superior for detecting MTC compared to OctreoScan-111 and give additional information on neuroendocrine tumours particularly if they are negative in somatostatin receptor scintigraphy (Endocr Relat Cancer. 2006 Dec;13(4):1203-11.; Eur J Nucl Med Mol Imaging. 2006 Nov;33(11):1273-9).

But due to the high kidney uptake the radiolabelled peptides could not be used for therapy. The high kidney uptake is correlated with the six negatively charged glutamic acids. 12 gastrin related compounds were designed, synthesised and compared as 111In labelled compounds. The best compounds with respect to a high tumour to kidney ratio are the minigastrins with six D-glutamic acids or six glutamines. These compounds still possess a methionine which can be oxidised easily. This is a disadvantage for clinical application because the receptor affinity is dramatically decreased after oxidation of the methionine and the production under GMP may be hampered dramatically.

A high potential for a significant improvement of the therapy and the image generation with patients having metastasized medullary thyroid carcinomas (MTC), small cell lung cancers (SCLC) and further CCK-2 receptor positive tumours has a specific labelling of the tumour cells with radio-labelled gastrin analogue. Basis for this finding is the proof of an over-expression of the respective CCK-2 target receptor at 920 of the investigated MTC, said proof being yielded by in-vitro studies [Reubi 1997]. Furthermore, the same working group identified the same over-expression of the CCK-2 target receptor at 570 of small cell lung cancers, 650 of astrocytomes and 1000 of stromal ovarial tumours.

First therapy studies (phase 0 study) had been executed at eight patients having advanced metastasized medullary thyroid carcinomas. For two patients a partial remission was achieved, four patients showed a stabilization of the formerly strongly progressive course of the cancer disease MTC after a therapy with ⁹⁰Y-labelled minigastrin analogue. This study had to be stopped due to the nephrotoxicity of the therapy in terms of a strong accumulation of the substances used in said assay in the kidneys.

With support of the European COST initiative (European Cooperation in Science and Technology), in the meantime a plurality of significantly improved radio-labelled gastrin-analogues have been synthesized by various working groups and have been investigated for their characteristics. As compared to the old gastrin analogue, these younger substances possess a significantly higher tumour-to-kidney ratio with respect to the absorption in human tissue (see US 2007/041903 A1, WO 2015/067473 A, Ludmila Melicharova et al: "Preclinical evaluation of gastrin derivatives labelled with sup111 /supin: radiolabelling, affinity profile and pharmacokinetics in rats", BIOMEDICAL PAPERS OF THE FACULTY OF MEDICINE OF PALACK UNIVERSITY, OLOMOUC CZECH REPUBLIC, 27 September 2013 (2013-09-27), ROOSENBURG S ET AL: "PET and SPECT Imaging of a Radio labeled Minigastrin Analogue Conjugated with DOTA, NOTA, and NODAGA and Labeled with Cu-64, Ga-68, and ln-111 ", MOLECULAR PHARMACEUTICS, vol. 11, no. 11, November 2014 (2014-11 ), pages 3930-3937).

Currently, out of these younger gastrin analogues, ¹⁷⁷Lu-PP-F11 (the linear minigastrin analogue with six D-Glu residues, hereinafter called PP-F11) exhibited best properties for future radio nuclide therapy due to its high favorable accumulation in the tumor accompanied by a low accumulation in the kidneys.

This PP-F11 minigastrin analogue has been further improved according to the international patent application WO 2015/067473 A1 wherein the minigastrin analogue PP-F11 having the formula: X-DGlu-DGlu-DGlu-DGlu-DGlu-DGlu-Ala-Tyr-Gly-Trp-Y-Asp-Phe-NH2, wherein Y stands for an amino acid replacing methionine and X stands for a chemical group attached to the peptide for the purpose of diagnostic and/or therapeutic intervention at CCK-2 receptor relevant diseases. In particular, very suitable compounds with respect to a high tumour to kidney ratio are minigastrin derivates with six D-glutamic acids or six glutamines. These compounds still possess a methionine which can be oxidised easily which is a disadvantage for clinical application under GMP due to the forms which may occur. Therefore, the replacement of the methionine by a non oxidizable isosteric amino acids but retaining the biological activity leads to a compound with no oxidation potential. This avoids the oxidation during storage and production which could be lead to lower affinity compound resulting in a low tumor to kidney ratio. In a preferred embodiment according to WO 2015/067473 A1, the methionine is replaced by norleucine. This so-called PP-F11N minigastrin exhibits excellent tumour-kidney-ratio and is therefore one very promising candidate for clinical applications.

It is therefore the objective of the present invention, as defined in the appended claims, to provide minigastrin derivates which further improve the accumulation by the metabolic stabilization of the peptide or improving its receptor affinity and specificity in CCK-2 receptor positive tumours by simultaneously very low accumulation in other organs, e.g. the kidneys.

This objective is achieved according to the present invention as claimed by a minigastrin derivate having the formula:
i) X-Z-Ala-Tyr-Gly-Trp-Met-Asp-Phe-NH₂ (Y), or
ii) DOTA-DGlu-DGlu-DGlu-DGlu-DGlu-DGlu- Ala-Tyr-Gly-Trp-Nle-Asp-Phe-NH₂, or
iii) DOTA-DGlu- Ala-Tyr-Gly-Trp-Met-Asp-Phe-NH₂,
wherein at least one of the connecting or terminal amide bonds between, before or after the amino acids of the sequence Z, Ala, Tyr, Gly, Trp, Met or Nle, Asp, Phe and NH₂ or Y (C-terminal) is replaced by a 1,4-disubstituted or a 1,5-disubstituted 1,2,3-triazole, while X stands for a chemical group attached to the peptide for the purpose of diagnostic and/or therapeutic intervention at CCK-2 receptor relevant diseases, Y stands for C-terminal modifications of the peptide, such as amide, primary and secondary amides, free carboxylic acids and carboxylic ester derivatives including but not limited to amides and esters derived from linear or branched alkyl-,alkenyl-, alkynyl- aromatic-, and heterocyclic alcohols, and Z stands for a linker or DGlu* wherein DGlu* stands for a chain of DGlu having 1 to 6 repetitions (-DGlu- to -DGlu-DGlu-DGlu-DGlu-DGlu-DGlu-).

These minigastrin derivates have a high receptor specific cell internalization, excellent IC₅₀ values towards CCK2 and sufficient plasma stability.

Preferably, the chelator for radiometals may be selected from a group consisting of DOTA (1,4,7,10-tetraazacyclododecane-1,4,7,10-tetraacetic acid), NOTA, NOTAGA, CHX-A"-DTPA and TCMC.

Further preferably, the radionuclide is selected from the group consisting of ¹⁷⁷Lu, ⁹⁰Y, ¹¹¹In, Ga-68/67, Tc-99m, Cu-64/67, Ac-225, Bi-213, Pb-212 and Th-227.

Further preferred embodiments of the present invention as claimed can be realized when DOTA-DGlu-DGlu-DGlu-DGlu-DGlu-DGlu-Ala-Tyr-Gly-Trp-Nle-Asp-Phe-NH2 is labelled with ¹⁷⁷Lu or DOTA-DGlu-Ala-Tyr-Gly-Trp-Nle-Asp-Phe-NH₂ is labelled with ¹⁷⁷Lu.

Preferred embodiments of the present disclosure are hereinafter described in more detail with respect to the attached drawings which depict in:

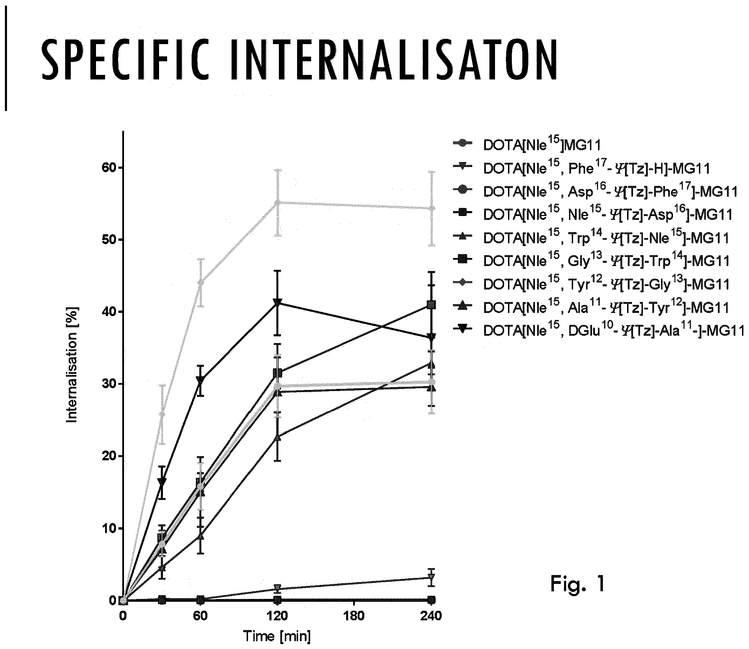

- Figure 1: the specific internalisation of [Nle¹⁵]-MG11 in its derivated forms according to the termed triazole-scan;

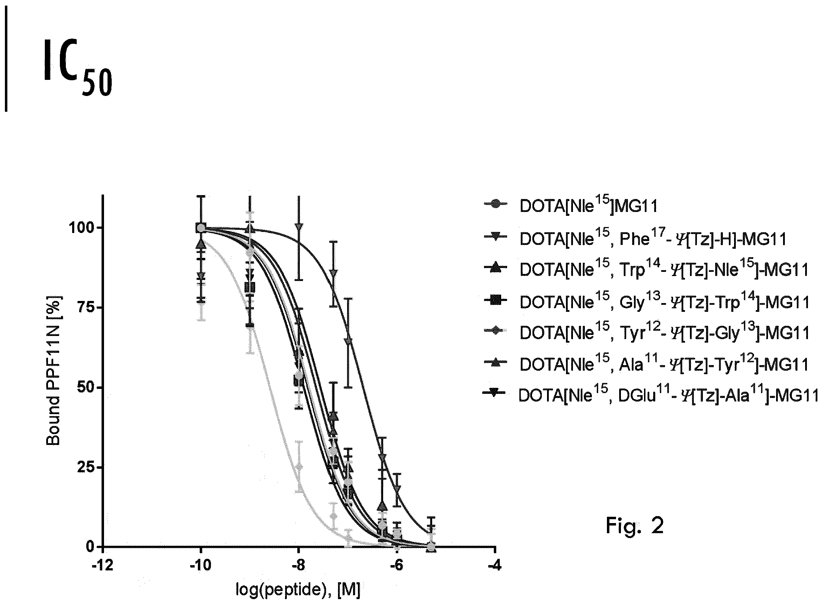

- Figure 2: the half maximal inhibitory concentration IC₅₀ of [Nle¹⁵]-MG11 in its derivated forms according to the termed triazole-scan;

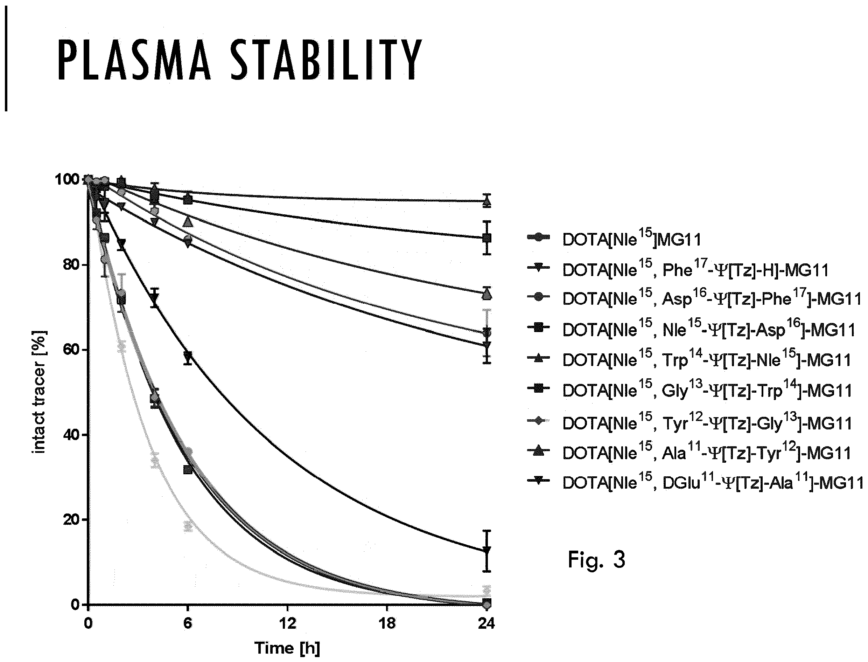

- Figure 3: the plasma stability of [Nle¹⁵]-MG11 in its derivated forms according to the termed triazole-scan;

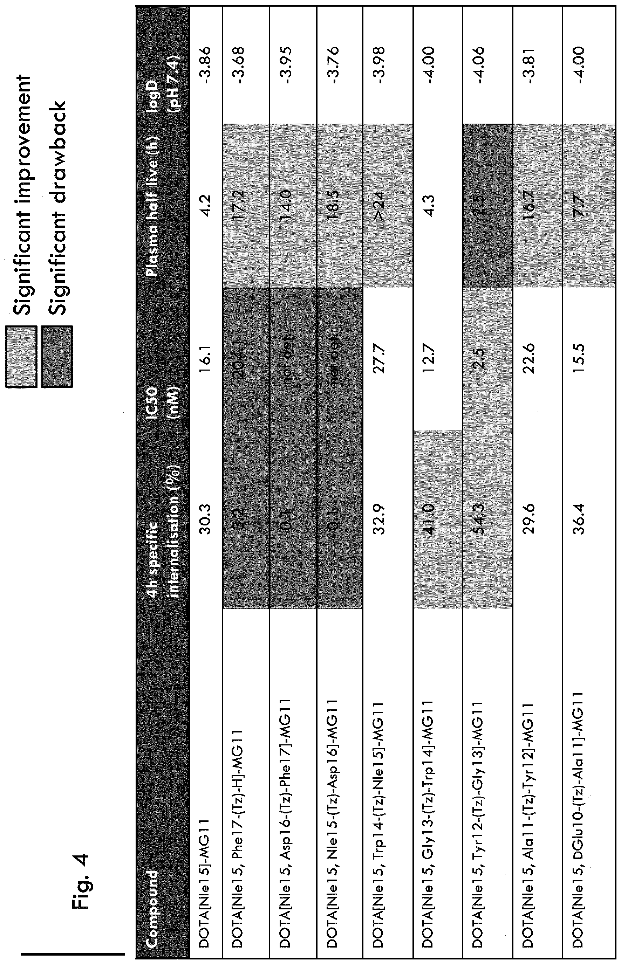

- Figure 4: the summary of the results shown in Figures 1 to 3;

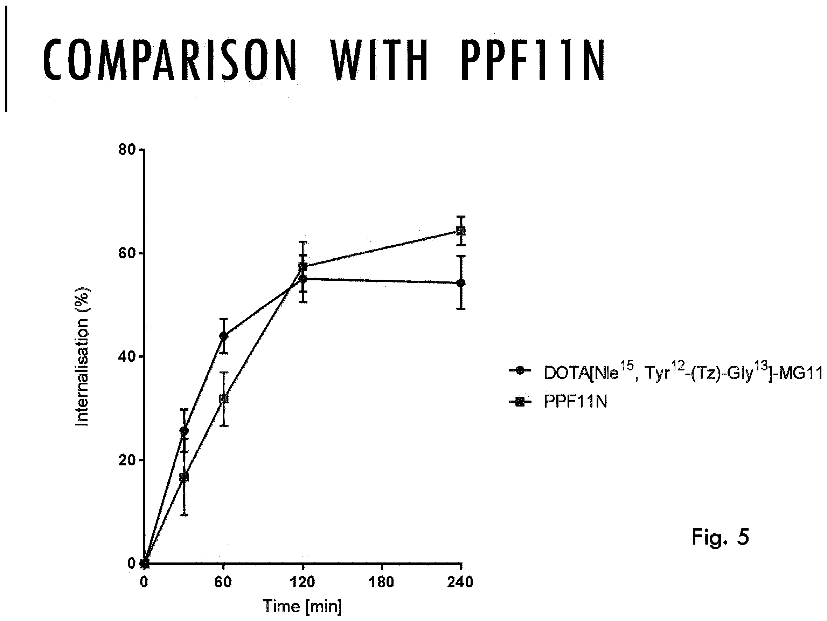

- Figure 5: a comparison of PP-F11N and the [Nle¹⁵]-MG11 derivate DOTA[Nle¹⁵, Tyr¹²-(Tz)-Gly¹³]-MG11;

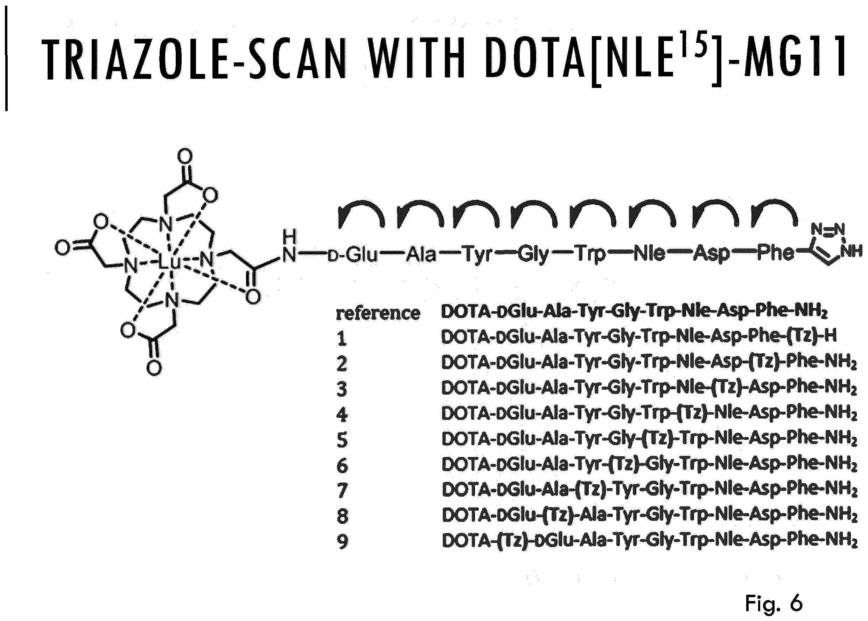

- Figure 6: principally the triazole scan with DOTA[Nle¹⁵]-MG11;

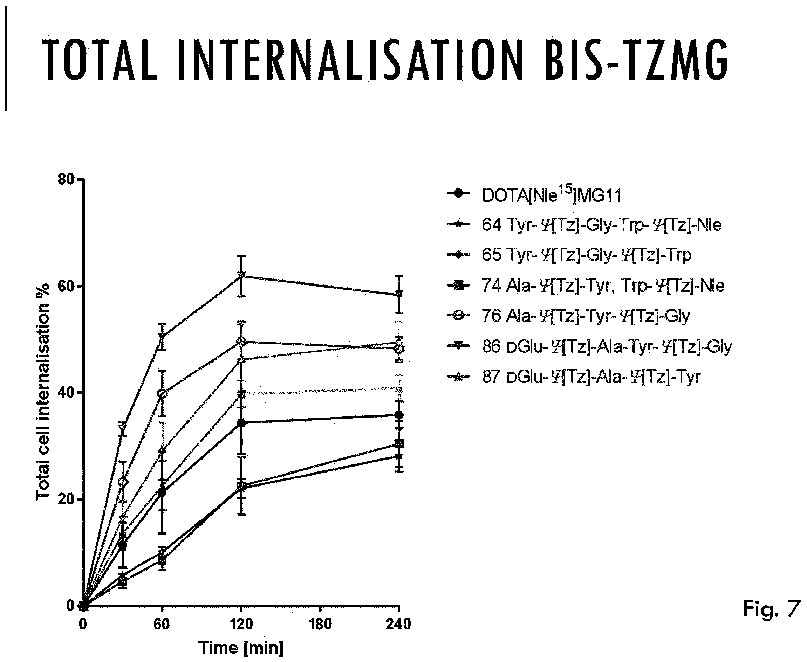

- Figure 7: the total internalisation of the Bis-TZMG from Table 2;

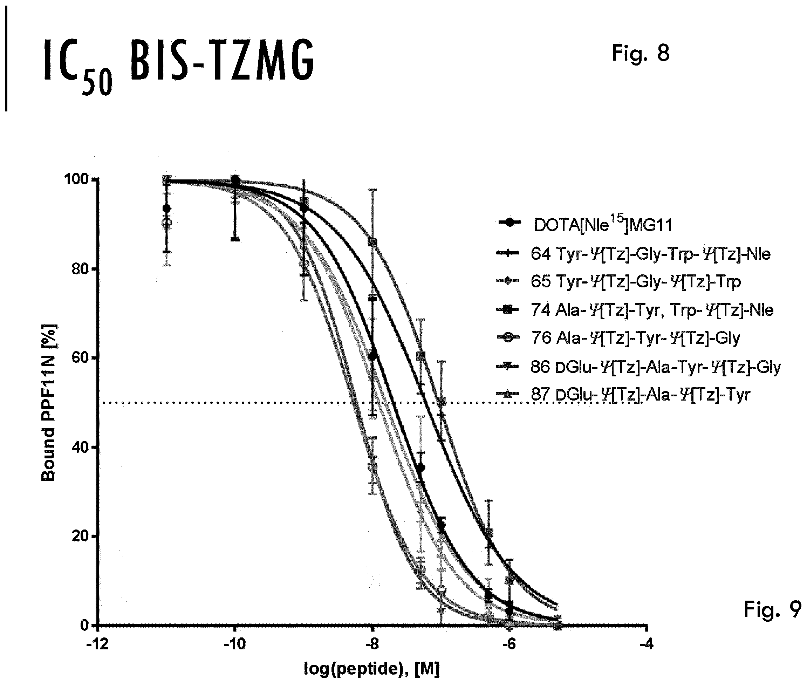

- Figure 8: the half maximal inhibitory concentration IC₅₀ of the Bis-TZMG from Table 2;

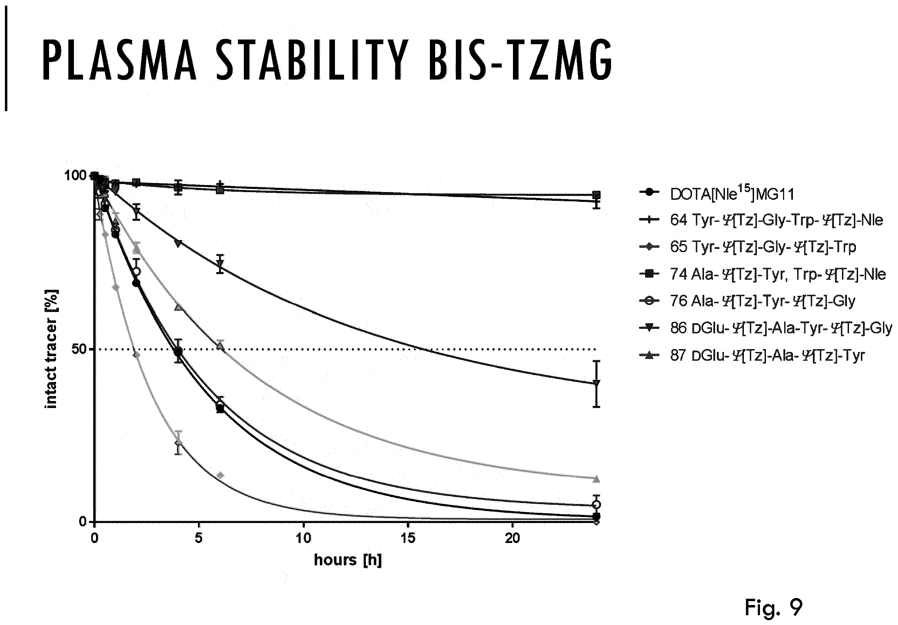

- Figure 9: the plasma stability of the Bis-TZMG from Table 2;

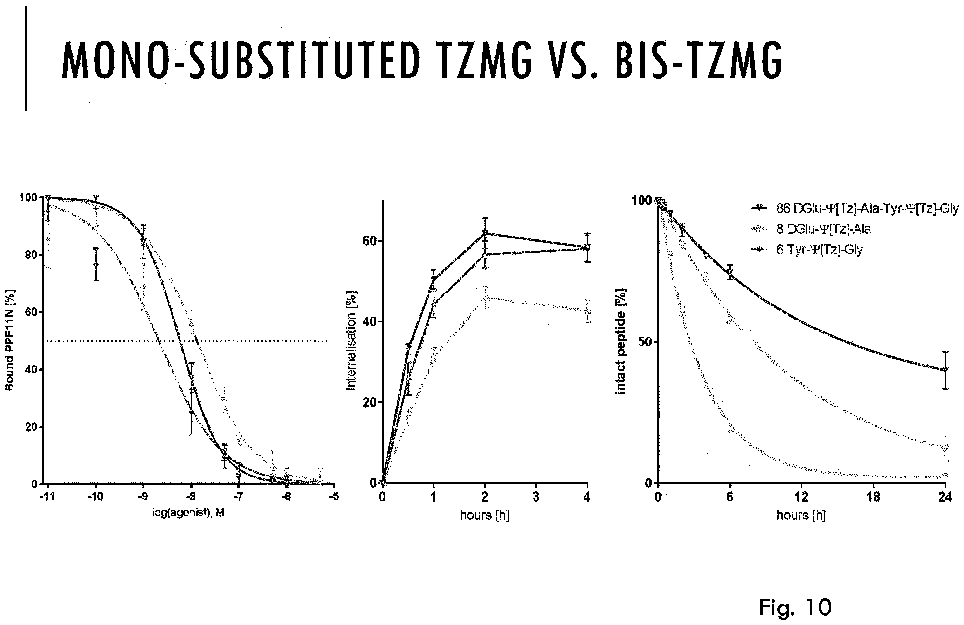

- Figure 10: in vitro characteristics of two mono-substituted triazolominigastrin vs. an analogue bearing both mutations showing additional improvement;

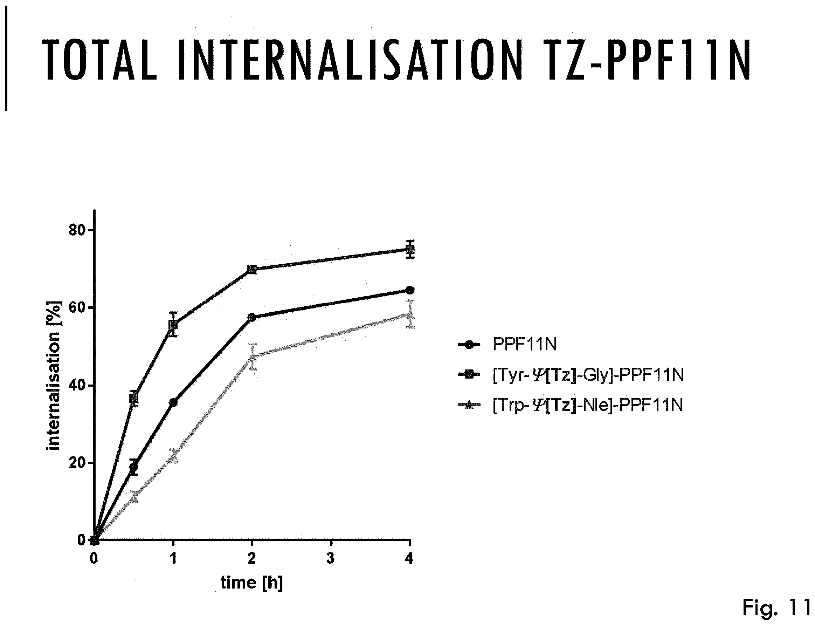

- Figure 11: the total internalisation of PPF11N and its derivated forms according to Table 3;

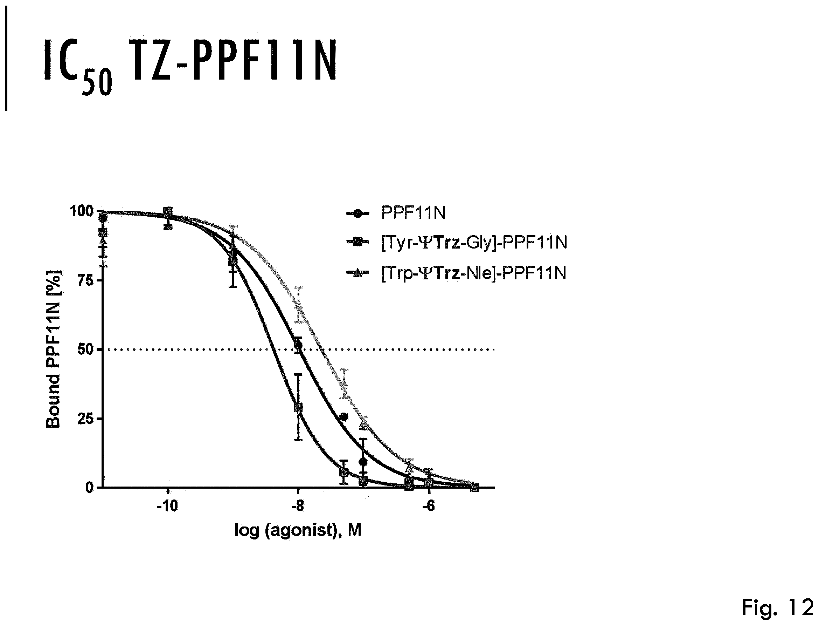

- Figure 12: the half maximal inhibitory concentration IC₅₀ of PPF11N in its derivated forms according to Table 3; and

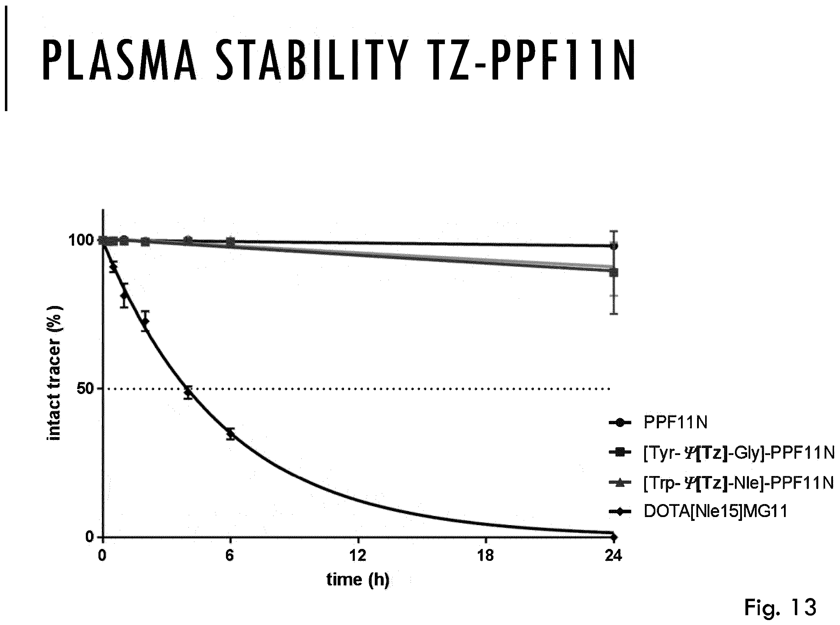

- Figure 13: the plasma stability of PPF11N in its derivated forms according to Table 3.

[Nle¹⁵]-MG11 is a truncated analogue of minigastrin, a regulatory peptide with high affinity and specificity towards the cholecystokinin 2 receptor (CCK2R) which is overexpressed in various types of cancer. The N-terminal conjugation of 1,4,7,10-tetraazacyclododecane-1,4,7,10-tetraacetic acid (DOTA) allows for radiolabelling of the peptide with metallic radionuclides (e.g., ¹⁷⁷Lu) and subsequent use for *in vivo* tumour imaging and peptide receptor radionuclide therapy.

A drawback of using MG11 as a tumour-targeting vector is its low tumour uptake due to fast enzymatic degradation which results in a biological half-life of only a few minutes. The systematic replacement of single amide bonds in the peptide sequence by stable 1,4-disubstituted or 1,5-disubstituted 1,2,3-triazoles, termed a triazole-scan, leads to an improved proteolytic stability and tumour targeting properties. To prove the general utility of this new

## Claims

1. A minigastrin derivate having the formula:
i) X-Z-Ala-Tyr-Gly-Trp-Met-Asp-Phe-NH₂ (Y), or
ii) DOTA-DGlu-DGlu-DGlu-DGlu-DGlu-DGlu- Ala-Tyr-Gly-Trp-Nle-Asp-Phe-NH₂, or
iii) DOTA-DGlu- Ala-Tyr-Gly-Trp-Met-Asp-Phe-NH₂,
wherein at least one of the connecting or terminal amide bonds between, before or after the amino acids of the sequence Z, Ala, Tyr, Gly, Trp, Met or Nle, Asp, Phe and NH₂ or Y (C-terminal) is replaced by a 1,4-disubstituted or a 1,5-disubstituted 1,2,3-triazole, while X stands for a chemical group attached to the peptide for the purpose of diagnostic and/or therapeutic intervention at CCK-2 receptor relevant diseases, Y stands for C-terminal modifications of the peptide, such as amide, primary and secondary amides, free carboxylic acids and carboxylic ester derivatives including but not limited to amides and esters derived from linear or branched alkyl-,alkenyl-, alkynyl- aromatic-, and heterocyclic alcohols, and Z stands for a linker or DGlu* wherein DGlu* stands for a chain of DGlu having 1 to 6 repetitions (-DGlu- to -DGlu-DGlu-DGlu-DGlu-DGlu-DGlu-).

2. The minigastrin derivate according to claim 1,
wherein the chelator for radiometals is selected from a group consisting of DOTA (1,4,7,10-tetraazacyclododecane-1,4,7,10-tetraacetic acid), NOTA, NOTAGA, CHX-A"-DTPA and TCMC.

3. The minigastrin derivate according to claim 2, wherein the radionuclide is selected from the group consisting of ¹⁷⁷Lu, ⁹⁰Y, ¹¹¹In, Ga-68/67, Tc-99m, Cu-64/67, Ac-225, Bi-213, Pb-212 and Th-227.

4. The minigastrin derivate according to claim 3, wherein DOTA-DGlu-DGlu-DGlu-DGlu-DGlu-DGlu-Ala-Tyr-Gly-Trp-Nle-Asp-Phe-NH2 is labelled with ¹⁷⁷Lu or DOTA-DGlu-Ala-Tyr-Gly-Trp-Nle-Asp-Phe-NH₂ is labelled with ¹⁷⁷Lu.

## Patentansprüche

1. Minigastrinderivat mit der Formel:
i) X-Z-Ala-Tyr-Gly-Trp-Met-Asp-Phe-NH₂ (Y) oder
ii) DOTA-DGlu-DGlu-DGlu-DGlu-DGlu-DGlu- Ala-Tyr-Gly-Trp-Nle-Asp-Phe-NH₂ oder
iii) DOTA-DGlu- Ala-Tyr-Gly-Trp-Met-Asp-Phe-NH₂,
wobei mindestens eine der verbindenden oder terminalen Amidbindungen zwischen, vor oder nach den Aminosäuren der Sequenz Z, Ala, Tyr, Gly, Trp, Met oder Nle, Asp, Phe und NH₂ oder Y (C-Terminus) durch ein 1,4-disubstituiertes oder ein 1,5-disubstituiertes 1,2,3-Triazol ersetzt ist, während X für eine chemische Gruppe steht, die an das Peptid zum Zweck der diagnostischen und/oder therapeutischen Intervention bei CCK-2-Rezeptor-relevanten Krankheiten gebunden ist, Y für C-terminale Modifikationen des Peptids steht, wie etwa Amid, primäre und sekundäre Amide, freie Carbonsäuren und Carbonsäureesterderivate, einschließlich jedoch nicht beschränkt auf Amide und Ester, die von linearen oder verzweigten Alkyl-, Alkenyl-, Alkinyl-, aromatischen und heterozyklischen Alkoholen abgeleitet sind, und Z für einen Linker oder DGlu* steht, wobei DGlu* für eine Kette von DGlu mit 1 bis 6 Wiederholungen (-DGlu- bis -DGlu-DGlu-DGlu-DGlu-DGlu-DGlu-) steht.

2. Minigastrinderivat nach Anspruch 1, wobei der Chelator für Radiometalle ausgewählt ist aus der Gruppe bestehend aus DOTA (1, 4, 7, 10-Tetraazacyclododecan-1, 4, 7, 10-tetraessigsäure), NOTA, NOTAGA, CHX-A"-DTPA und TCMC.

3. Minigastrinderivat nach Anspruch 2, wobei das Radionuklid ausgewählt ist aus der Gruppe bestehend aus ¹⁷⁷Lu, ⁹⁰Y, ¹¹¹In, Ga-68/67, Tc-99m, Cu-64/67, Ac-225, Bi-213, Pb-212 und Th-227.

4. Minigastrinderivat nach Anspruch 3, wobei DOTA-DGlu-DGlu-DGlu-DGlu-DGlu-DGlu-Ala-Tyr-Gly-Trp-Nle-Asp-Phe-NH2 mit ¹⁷⁷Lu markiert ist oder DOTA-DGlu-Ala-Tyr-Gly-Trp-Nle-Asp- Phe-NH₂ mit ¹⁷⁷Lu markiert ist.

## Revendications

1. Dérivé de minigastrine ayant la formule :
i) X-Z-Ala-Tyr-Gly-Trp-Met-Asp-Phe-NH₂ (Y) ou
ii) DOTA-DGlu-DGlu-DGlu-DGlu-DGlu-DGlu-Ala-Tyr-Gly-Trp-Nle-Asp-Phe-NH₂ ou
iii) DOTA-DGlu-Ala-Tyr-Gly-Trp-Met-Asp-Phe-NH₂,
au moins une des liaisons amide de connexion ou terminales entre, avant ou après les acides aminés de la séquence Z, Ala, Tyr, Gly, Trp, Met ou Nle, Asp, Phe et NH₂ ou Y (C-terminal) étant remplacé par un 1,2,3-triazole 1,4-disubstitué ou 1,5-disubstitué, alors que X correspond à un groupe chimique attaché au peptide pour l'objectif de diagnostic et/ou d'intervention thérapeutique aux maladies concernant le récepteur CCK-2, Y correspond aux modifications C-terminales du peptide, telles qu'un amide, des amides primaires et secondaires, des acides carboxyliques libres et des dérivés d'ester carboxylique comprenant mais non limités à des amides et des esters dérivés d'alcools alkyle-, alcényle-, alcynyle-, aromatique- et hétérocylique linéaires ou ramifiés et Z correspond à un lieur ou DGlu* DGlu* correspondant à une chaîne de DGlu ayant 1 à 6 répétitions (-DGlu- à -DGlu-DGlu-DGlu-DGlu-DGlu-DGlu-).

2. Dérivé de minigastrine selon la revendication 1,
dans lequel le chélateur pour radiométaux est choisi dans un groupe constitué par DOTA (acide 1,4,7,10-tétraazacyclododécane-1,4,7,10-tétraacétique), NOTA, NOTAGA, CHXA''-DTPA et TCMC.

3. Dérivé de minigastrine selon la revendication 2, dans lequel le radionucléide est choisi dans le groupe constitué par ¹⁷⁷Lu, ⁹⁰Y, ¹¹¹In, Ga-68/67, Tc-99m, Cu-64/67, Ac-225, Bi-213, Pb-212 et Th-227.

4. Dérivé de minigastrine selon la revendication 3, dans lequel DOTA-DGlu-DGlu-DGlu-DGlu-DGlu-DGlu-Ala-Tyr-Gly-Trp-Nle-Asp-Phe-NH₂ est marqué avec ¹⁷⁷Lu ou DOTA-DGlu-Ala-Tyr-Gly-Trp-Nle-Asp-Phe-NH₂ est marqué avec ¹⁷⁷Lu.
